# EUROPÄISCHE PATENTSCHRIFT

(11) **EP 2 885 259 B1**
(45) Veröffentlichungstag und Bekanntmachung des Hinweises auf die Patenterteilung: **07.10.2020**
(21) Anmeldenummer: 13752623.2
(22) Anmeldetag: 20.08.2013
(51) Int. Cl.: A61L 27/10, A61L 27/12, A61L 27/50, A61K 6/802, A61K 6/818, A61K 6/822, A61K 6/824, C04B 35/488, C04B 35/626, C04B 35/645, A61L 27/42, A61F 2/30

(54) **ZIRKONOXID-BASIERTER VERBUNDWERKSTOFF**
ZIRCONIUM OXIDE-BASED COMPOSITE MATERIAL
MATÉRIAU COMPOSITE À BASE D'OXYDE DE ZIRCONIUM

(30) Priorität: 20.08.2012 DE 102012214749; 04.09.2012 DE 102012215658
(43) Veröffentlichungstag der Anmeldung: 24.06.2015
(73) Patentinhaber: CeramTec GmbH, 73207 Plochingen (DE)
(72) Erfinder: GOTTWIK, Lukas, 73092 Heiningen (DE); KUNTZ, Meinhard, 73733 Esslingen (DE); PORPORATI, Alessandro, Alan, 73728 Esslingen (DE); EHRLICH, Juliane, 70327 Stuttgart (DE); MORHARDT, Andreas, 73730 Esslingen (DE); FRIEDERICH, Kilian, 73207 Plochingen (DE)
(74) Vertreter: Fehrenbacher, Eckhard Anton
(86) Internationale Anmeldenummer: PCT/EP2013/067281
(87) Internationale Veröffentlichungsnummer: WO 2014/029757

(56) Entgegenhaltungen:
- EP-A1- 0 548 948
- EP-A1- 2 377 506
- WO-A1-2008/040813
- WO-A1-2011/083023
- WO-A2-2013/004361
- DE-A1- 4 020 893
- DE-A1- 19 540 452
- DE-A1-102008 042 015
- US-A1- 2007 049 484
- SHEN Z ET AL: "DENSE HYDROXYAPATITE-ZIRCONIA CERAMIC COMPOSITES WITH HIGH STRENGTH FOR BIOLOGICAL APPLICATIONS", ADVANCED MATERIALS, WILEY VCH VERLAG, DE, Bd. 13, Nr. 3, 5. Februar 2001 (2001-02-05), Seiten 214-216, XP001008957, ISSN: 0935-9648, DOI: 10.1002/1521-4095(200102)13:3<214::AID-ADM A214>3.0.CO;2-5

## Beschreibung

Die Erfindung betrifft einen keramischen Verbundwerkstoff, seine Herstellung und Verwendung. Insbesondere betrifft die Erfindung einen Zirkonoxid-basierten Verbundwerkstoff, eine homogene, mehrphasige, biokompatible und polykristalline Keramik.

Der Zirkonoxid-basierten Verbundwerkstoff kann im dentalen Sektor bei der Herstellung von Brücken und Kronen verwendet werden, beispielsweise bei der Herstellung von Zahnimplantaten, bei der Herstellung von medizintechnischen Bauteilen wie z.B. Wirbelsäulenimplantaten, aber auch allgemein in Bereichen, in denen eine technische Keramik mit schädigungsfreier Hartbearbeitbarkeit benötigt wird, wie z.B. beim maschinellen Bearbeiten wie Schneiden, Fräsen und Bohren.

Keramische Werkstoffe besitzen aufgrund der chemischen Beständigkeit, der mechanischen und physikalischen Eigenschaften, sowie der optischen Eigenschaften, die eine exzellente Ästhetik zulassen, im dentalen Markt Vorteile gegenüber herkömmlichen metallischen Werkstoffen.

Der allgemeine Trend bei Dentalkeramiken geht in Richtung "vollkeramische Systeme". Dennoch werden heute noch häufig Keramiken als Verblendungen auf metallischen Gerüsten aufgebracht. Dentalkeramiken lassen sich anhand ihrer Herstellungsmethode und ihrer kristallinen Phase klassifizieren.

Metall-keramische Systeme gibt es seit 1960. Um eine ästhetisch akzeptable Restauration in Anlehnung an den natürlichen Zahn zu erhalten, wird eine Verblendkeramik auf ein Metallgerüst aufgebracht. Typische Verblendmaterialien bestehen aus feldspatischen Gläsern, gewöhnlich Leuzitkristall-basiert. Die Zugabe von Leuzitkristallen (KAlSi₂O₆) in das feldspatische Glasgefüge führt zu optimalen Eigenschaften hinsichtlich der thermischen Ausdehnungskoeffizienten von Gerüst und Verblendung. Leuzitkristalle bilden sich durch inkongruentes Schmelzen von natürlichem Feldspat bei Temperaturen zwischen 1150 und 1530°C. Durch die Variation des Leuzitkristallgehalts im Glas kann der thermische Ausdehnungskoeffizient gezielt gesteuert und an das metallische Gerüst angepasst werden. Gewöhnlich beträgt der typische Leuzitkristallgehalt in feldspatischem Glas zwischen 15 und 25 Vol.-%. Somit ist der thermische Ausdehnungskoeffizient geringer als der des Metalls, und die aufgebrachte Verblendung wird unter Druck gesetzt. Klassisch werden Verblendkeramiken unter Vakuum gesintert, um die Porosität im Endprodukt zu reduzieren. Die mechanischen Eigenschaften der Leuzitkristall-basierten Gläser (auch Dentalporzellan genannt) sind aufgrund der Glasphase die geringsten von allen keramischen Werkstoffen, die in der Zahnmedizin verwendet werden. Bis 2005 wurden noch 50% aller Zahnrestaurationen mit metall-keramischen Systemen hergestellt.

Vollkeramische Systeme sind metallfrei und seit 30 Jahren verfügbar. Die Prozesstechnik wird ständig weiterentwickelt (z.B. Heißpressen, Schlickergießen, CAD/CAM Bearbeitung). Der Hauptunterschied zu den metall-keramischen Systemen ist ein weit höherer kristalliner Phasenanteil, der zwischen 35 und 100 Vol.-% liegen kann. Die mechanischen Eigenschaften verbessern sich, aber auch die Opazität nimmt zu, was hinsichtlich der geforderten Ästhetik nachteilig ist.
EP 2 377 506 A1 beschreibt einen Verbundwerkstoff für die Dentaltechnik aus einem CeO₂-stabilisiertem Zirkonoxid und Aluminat. WO 2008/040813 A1 beschreibt einen Verbundwerkstoff aus Y₂O₃ und CeO₂-stabilisiertem Zirkonoxid, dessen Sekundärphase Lanthanaluminat umfasst. DE 195 40 452 A1 zeigt Verbundwerkstoffe auf Zirkonoxidbasis mit Aluminiumoxid-Sekundärphase, die beständig gegen hydrothermale Alterung sind. WO 2011/083023 A1 betrifft einen Verbundwerkstoff der Medizintechnik, der aus Zirkonoxid, Aluminiumoxid und Dispersoiden besteht. DE 10 2008 042015 A1 zeigt eine zweiphasenverstärkte keramische Zusammensetzung von Mullit in Zirkonoxid. US 2007/049484 A1 zeigt einen Verbund aus keramischen Nanopartikeln in einer Matrix aus Zirkonoxid. Die Nanopartikel bestehen aus Siliziumoxid, Zirkon oder Mullit. DE 40 20 893 A1 zeigt eine Dispersion von Calciumphospatkristallen in einer Matrix aus Zirkonoxid als Implantationsmaterial für künstliche Knochen und Dentalimplantate. SHEN et al. ADV MAT (2001) S. 214-216 zeigt einen dichten Hydroxylapatit-Zirkonoxid-Verbundwerkstoff für biologische Anwendungen, wie z.B. Implantate. EP 0 548 948 A1 zeigt einen gesinterten Körper mit einer Zirkonoxid-Matrix und darin dispergiertem Aluminiumoxid.

Es gibt eine Vielzahl von Faktoren, die auf die Langlebigkeit der vollkeramischen Systeme einen Einfluss haben, z.B. orales Umgebungsmilieu, schwankende pH-Werte von sauer bis basisch, zyklische Belastung und extreme Belastungsspitzen während des Kauens. Vollkeramische Systeme mit höheren Glasphasenanteilen zeigen häufig Spannungsrisskorrosion als Versagensursache. Aufgrund der hydrothermalen Alterung von Y-TZP-Keramiken (100 Vol.-% kristalline Phase, Y-stabilisiertes tetragonales Zirkonoxid) bei niedrigen Temperaturen werden Tests in Normen verlangt, bei denen die Langlebigkeit in menschlicher Umgebung und unter zyklischer Belastung bewertet werden soll.

Vollkeramische Systeme werden hauptsächlich aufgrund der Herstellungsmethode klassifiziert (z.B. Heißpressen, Trockenpressen und Sintern, Schlickergießen, CAD/CAM Bearbeitung). Beim Heißpressen wurden zuerst Leuzitkristall-basierte Gläser mit einem kristallinen Phasenanteil zwischen 35 und 45 Vol.-% verwendet. Die mechanischen Eigenschaften sind um den Faktor 2 gegenüber den Leuzitkristall-basierten Gläsern der metall-keramischen Systeme höher. Mehrmaliges Aufheizen kann dabei die Leuzitkristallisation begünstigen und zu höheren Festigkeiten führen.

Heute wird eine neue Glaskeramik für das Heißpressen verwendet. Das Material besteht aus einem Lithiumdisilikat-basierten Glas mit einem kristallinen Phasenanteil von 65 Vol.-%. Röntgenographische Untersuchungen haben neben Lithiumdisilikat (Li₂Si₂O₅) weitere Kristallphasen wie Lithiummetasilikat (Li₂SiO₃) und Cristobalit (SiO₂) gezeigt. Die mechanischen Eigenschaften sind im Vergleich zu den Leuzitkristall-basierten Gläser nochmals um den Faktor 2 höher.

Das Trockenpressen und Sintern von vollkeramischen Systemen wird seit den frühen 90iger Jahren verwendet. Die Herstellung erfolgt computerunterstützt und berücksichtigt die Sinterschwindung des Presslings beim Sintern. Verwendet werden Aluminiumoxid- und Zirkonoxid-basierte Keramiken (100 Vol.-% kristalliner Phasenanteil) als Gerüstmaterial, auf welchen zusätzlich eine Verblendung aus Glaskeramik aufgebracht wird. Aluminiumoxidkeramiken zeichnen sich durch eine Biegefestigkeit von ca. 600 MPa und durch ein exzellentes in-vivo Verhalten aus.

Das Schlickergießen wird seit den 90iger Jahren angewendet. Dabei wird ein poröser Grünkörper mittels Schlickerguss aus kristallinen Phasen hergestellt, anschließend gesintert und mit einem Lanthan-basiertem Glas infiltriert.

Auf dem Dentalmarkt sind folgende Glaskeramiken erhältlich:
Aluminiumoxid (Al₂O₃), Spinell (MgAl₂O₄) oder 12Ce-TZP/Al₂O₃-Komposit. Das glasinfiltrierte Aluminiumoxid hat vergleichbare mechanische Eigenschaften zur Lithiumdisilikat-basierten Glaskeramik, jedoch eine minimal höhere Opazitat. Der glasinfiltrierte Spinell besitzt deutlich mehr Transluzenz und vergleichbare mechanische Eigenschaften zu Lithiumdisilikat-basierten Glaskeramik. Das glasinfiltrierte Zirkonoxid/Aluminiumoxid-Komposit zeigt die höchsten Festigkeiten und Risszähigkeiten aller schlickergegossenen Dentalkeramiken.

Die computergesteuerte CAD/CAM Bearbeitung von keramischen Blöcken bzw. Blanks erfolgt seit den frühen 70iger Jahren und wurde von Duret eingeführt. Damals erfolgte die Bearbeitung an dichtgesinterten Blanks. Heute wird hauptsächlich mit vorgesinterten Blanks gearbeitet.

Glaskeramik eignet sich für die CAD/CAM-Bearbeitung im dichtgesinterten Zustand aufgrund der sehr guten Bearbeitbarkeit. Früher wurden typische Glimmerkristallbasierte Gläser aufgrund der idealen Bearbeitbarkeit verwendet. Heute werden feldspatische Gläser mit Sanidin-, Leuzit- oder Lithiumdisilikatkristallen verwendet. Allerdings zeigt die CAD/CAM-Bearbeitung an dichtgesinterten Glaskeramiken einen signifikanten Werkzeugverschleiß. Oberflächenfehler können das in-vivo Verhalten negativ beeinflussen.

Allgemein sind Glaskeramiken sehr gut bearbeitbar. Aufgrund unterschiedlicher thermischer Ausdehnungskoeffizienten von Kristall und Glasmatrix entstehen während des Abkühlens Mikrorisse entlang der Phasengrenzen. Zusätzlich besitzen die kristallinen Phasen eine sehr gute Spaltbarkeit entlang der Längsausrichtung (vor allem Glimmer entlang der kristallographischen (001)-Ebene). Die Kristallphasen sollten keine Vorzugsorientierung haben, d.h. isotrop im Glasgefüge verteilt sein. Ein durch ein Werkzeug eingebrachter Riss verläuft entlang der Spaltebene oder auch entlang der Phasengrenze zwischen Kristall und Glasmatrix. Dadurch wird der Riss während der Bearbeitung ständig abgelenkt, und es werden nur kleine Bereiche der Oberfläche aus dem Werkstück herausgebrochen.

Seit 2001 erfolgt die CAD-CAM-Bearbeitung an vorgesinterten Zirkonoxid-Blanks. Die Bearbeitung ist leichter, schneller und zeigt einen geringeren Werkzeugverschleiß als wenn dichtgesinterte Blöcke bearbeitet werden müssten. Jedoch müssen die gefertigten Werkstücke anschließend dicht gesintert werden. Schwankungen in der Sinterschwindung einhergehend mit Maßabweichungen, sowie Nachbesserungsarbeiten per Hand vom Zahntechniker führen zu einem erhöhten Schädigungsrisiko des Zirkonoxids.

Zirkonoxid als Gerüstmaterial besitzt bisher die besten mechanischen Eigenschaften. Es treten aber häufig aufgrund der zusätzlich benötigten Verblendkeramik am Interface zwischen Gerüst und Verblendung Risse durch Phasenumwandlung der tetragonalen Zirkonoxidphase auf. Es gibt bereits seit einiger Zeit mehrere, bereits veröffentliche 3-Jahres- und 5-Jahres-Invivo-Studien. Das Fazit der Studien ist eine exzellent Erfolgsrate, aber mit geringer Überlebensrate bei Komplikationen wie z.B. Kariesbefall oder Abplatzungen der Verblendung. Der aktuelle Entwicklungstrend geht eindeutig Richtung Zirkonoxid/Aluminiumoxid-Verbundwerkstoffe mit den Zielen, die hydrothermale Alterungsbeständigkeit und mechanischen Eigenschaften zu verbessern.

Die Aufgabe der Erfindung besteht nun darin, die Nachteile des Stands der Technik zu vermeiden und insbesondere eine Keramik bereitzustellen, deren mechanische Eigenschaften eine schädigungsfreie Hartbearbeitung zulässt und/oder die eine gute hydrothermale Alterungsbeständigkeit aufweist. Darüber hinaus sollte die Keramik mit den gängigen Verfahren herstellbar und bearbeitbar sein. Die Lösung dieser Aufgabe erfolgt mittels eines Verbundwerkstoffs gemäß Anspruch 1 und einem zugehörigen Verfahren gemäß Anspruch 7.

Die Vorteile des erfindungsgemäßen neuen Werkstoffs gegenüber dem Stand der Technik werden anhand der verbesserten "Schädigungstoleranz" quantitativ erfasst. Schädigungstoleranz ist ein mechanischer Kennwert, der den Widerstand eines Werkstoffs gegen eine von außen aufgebrachte Schädigung beschreibt. Die Schädigung kann in der Praxis beispielsweise durch eine Schleifbearbeitung mit diamantbesetzten Werkzeugen erfolgen.

Für die Messung der Schädigungstoleranz im Labor wird auf den Prüfkörper mittels einer Diamantspitze (Vickers) unter einer definierten Beanspruchungskraft eine Schädigung aufgebracht. Im Bereich des Härteeindrucks bilden sich Risse aus, so dass der Prüfkörper an dieser Stelle eine Schwächung erfährt. Die Schwächung wird quantitativ durch Messung der Rest-Bruchspannung, bzw. Restfestigkeit an dieser Stelle ermittelt. Je höher die Restfestigkeit nach einer definierten Schwächung, um so höher ist die Schädigungstoleranz des Werkstoffs.

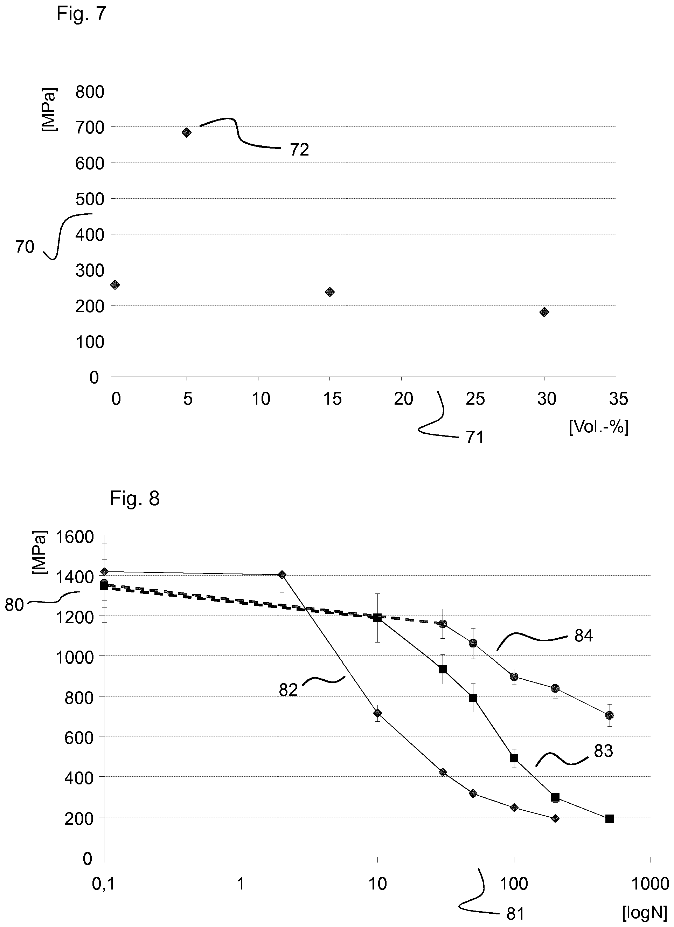

Zur detaillierten Beschreibung der Schädigungstoleranz werden an einer Serie von Prüfkörpern Schädigungen mit unterschiedlichen Beanspruchungskräften aufgebracht. Dadurch ergibt sich eine Kennlinie für den Werkstoff (Restfestigkeit vs. Beanspruchungskraft). Der Nachweis einer verbesserten Schädigungstoleranz eines Werkstoffs gegenüber dem Stand der Technik erfolgt durch Vergleich dieser Kennlinien, siehe Figs. 7 und 8.

Die Erfindung betrifft die Herstellung und die Verwendung eines Zirkonoxid-basierten Verbundwerkstoffs, insbesondere für eine schädigungsfreie Hartbearbeitung im dichtgesinterten Zustand. Die Herstellung des erfindungsgemäßen Verbundwerkstoffs erfolgt mittels an sich bekannter, konventioneller Keramiktechnologie. Die wesentlichen Prozessschritte sind beispielsweise:
a) Pulvermischung gemäß vorgegebener Zusammensetzung in Wasser ansetzen; ggf. Verwendung von Verflüssigern zur Vermeidung der Sedimentation
b) Homogenisierung im Dissolver (schnelllaufender Rührer)
c) Mahlen in Rührwerkskugelmühle, dabei Erhöhung der spezifischen Oberfläche der Pulvermischung (=Zerkleinerung und Homogenisierung)
d) ggf. Zugabe von organischen Bindern
e) Sprühtrocknen, dabei entsteht ein rieselfähiges Granulat mit definierten Eigenschaften
f) Befeuchten des Granulats mit Wasser und ggf. weiteren Presshilfsmitteln
g) Axialpressen von Blöcken
h) Spanabhebende Bearbeitung von Blöcken im Grün- oder vorgesinterten Zustand, dabei wird unter Berücksichtigung der Sinterschwindung weitgehend die Endkontur abgebildet.
i) Sinterung (Dies kann auch in einer 3-stufigen Sinterung erfolgen: Vorbrand auf eine theoretische Dichte von ca. 97%. Die noch verbleibenden Restporen sind nach außen geschlossen. Heißisostatisches Pressen (HIP) unter hoher Temperatur und hohem Gasdruck, dadurch praktisch vollständige Endverdichtung. Sogenannter Weißbrand, dadurch wird das beim heißisostatischen Pressen erzeugte Ungleichgewicht der Sauerstoffionen in der Keramik ausgeglichen.)
j) Hartbearbeitung durch Schleifen und Polieren mit diamantbesetztem Werkzeug.

Verwendet werden kann der erfindungsgemäße Verbundwerkstoff beispielsweise zur Herstellung von Sinterformkörpern, zur Herstellung von künstlichem Zahnersatz, Zahnrestaurationen wie Brücken, Kronen, Inlays und Onlays, zur Herstellung von Zahnwurzelstiften, Implantaten und Abutments. Bevorzugt wird die Anwendung im Bereich der Implantattechnik. Besonders bevorzugt ist die Anwendung im Wirbelsäulenbereich als Spacer bzw. Cage.

Der Zirkonoxid-basierte Verbundwerkstoff besitzt als keramische Matrix Zirkonoxid und darin dispergiert zumindest eine Sekundärphase bzw. Dispersoid und ggf. weitere Zuschlagstoffe. Der Verbundwerkstoff umfasst als erste Phase einen Zirkonoxidanteil von mindestens 51 Vol.-% und eine Sekundärphase mit einem Anteil von 1 bis 49 Vol.-% und gegebenenfalls einen oder mehrere anorganische Zuschlagstoffe. Das Zirkonoxid liegt, bezogen auf den Gesamt-Zirkonoxidanteil, zum überwiegenden Teil, d.h. zu 90 bis 99 %, bevorzugt zu 95 bis 99%, in der tetragonalen Phase vor, wobei die Stabilisierung der tetragonalen Phase des Zirkonoxids zum Teil nicht nur chemisch, sondern auch mechanisch erfolgt. Die Begriffe "Sekundärphase" und "Dispersoid" werden im Rahmen dieses Textes als Synonyme verwendet.

Die zusätzlich zur chemischen Stabilisierung vorliegende mechanische Stabilisierung des Zirkonoxids in der tetragonalen Phase ermöglicht es vorteilhaft, den Gehalt an chemischen Stabilisatoren im Vergleich zum Stand der Technik zu verringern.

Die mechanische Stabilisierung des Zirkonoxids in der tetragonalen Phase ist aus ZTA-Verbundwerkstoffen (zirkonia toughened alumina) bekannt. Dort wurde davon ausgegangen, dass die mechanische Stabilisierung zum einen durch die Korngröße des Zirkonoxids beeinflusst wird. Diese sollte in Aluminiumoxid-Verbundwerkstoffen nicht größer als 0,5 µm, gemessen nach dem Linienschnitt-Verfahren, sein. Andererseits ging man davon aus, dass die Einbettung der einzelnen Zirkonoxidpartikel in die Aluminiumoxid-Matrix einen wesentlichen Anteil an der mechanischen Stabilisierung hat, wobei Mindestgehalte an Aluminiumoxid von 65 Vol.-%, vorzugsweise höhere Gehalte, als notwendig erachtet wurden.

Mit der vorliegenden Erfindung konnte nachgewiesen werden, dass eine solche mechanische Stabilisierung des Zirkonoxids nicht nur funktioniert, wenn Zirkonoxid als dispersoide Phase in Aluminiumoxid eingelagert wird, sondern auch in einer Keramik, die im Wesentlichen aus Zirkonoxid besteht. Die erfindungsgemäße Zugabe der Sekundärphasen/Dispersoiden gleicht die bei der Umwandlung der tetragonalen Kristallstruktur in die monokline Kristallstruktur des Zirkonoxids auftretende Dehnung aus, indem Mikrobewegungen/-scherungen auf Kristallit-Ebene in einem ansonsten vergleichsweise starren keramischen Gefüge möglich werden, ohne dass notwendig makroskopische Risse entstehen.

Weiterhin wird unter mechanischer Stabilisierung auch verstanden, dass die tetragonale Kristallphase des Zirkonoxids durch mechanische Spannungen im Gesamtgefüge stabilisiert wird. Unterschiedliche Wärmeausdehnungskoeffizienten von ZrO₂ und Sekundärphase beim Abkühlen nach dem Sinterprozess können zu solchen Spannungen führen.

Die mechanische Stabilisierung ist insbesondere deshalb vorteilhaft, weil sie dazu führt, dass geringere Anteile an Verbindungen, die zur chemischen Stabilisierung verwendet werden, notwendig sind. Die chemische Stabilisierung beruht auf der partiellen Substitution von Zirkonoxid-Ionen gegen Kationen, die im Kristallgitter Sauerstoffleerstellen erzeugen und somit einen geringeren "Platzbedarf" aufweisen. Die Sauerstoffleerstellen im Gitter können jedoch Angriffspunkte für eine hydrothermale Alterung sein. Somit führt eine mechanische Stabilisierung, die gleichzeitig die Notwendigkeit für eine chemische Stabilisierung zumindest vermindert, zu einer verbesserten Beständigkeit des Verbundwerkstoffs gegenüber hydrothermaler Alterung.

Die Zirkonoxidmatrix besitzt eine Korngröße von durchschnittlich 0,1 bis 2,0 µm, und bevorzugt von durchschnittlich 0,5 bis 2,0 µm. Der Anteil an chemischen Stabilisatoren im erfindungsgemäßen Verbundwerkstoff (Anteil jeweils relativ zum Zirkonoxid-Gehalt) beträgt für Y₂O₃ ≤ 3 mol-%, bevorzugt ≤ 2,5 mol-%, und gemäß einer Weiterbildung der Erfindung für CeO₂ ≤ 12 mol-%, für Gd₂O₃ ≤ 3 mol-%, für Sm₂O₃ ≤ 3 mol-% und für Er₂O₃ ≤ 3 mol-%. Die chemischen Stabilisatoren im erfindungsgemäßen Verbundwerkstoff umfassen einen oder mehrere der genannten Zuschlagstoffe, wobei Y₂O₃ enthalten ist. Der Gesamtgehalt an chemischen Stabilisatoren beträgt vorteilhaft < 12 mol-% relativ zum ZrO₂-Gehalt.

Das Zirkonoxid und das Dispersoid können gemäß einer Ausführungsform lösliche Bestandteile enthalten. Lösliche Bestandteile können z.B. Cr, Fe, Mg, Ca, Ti, Y, Ce, Lanthaniden und/oder V sein. Diese Bestandteile können zum einen als Farbadditive und zum anderen als Sinterhilfsmittel fungieren. Sie werden in der Regel als Oxide zugegeben.

Die Partikelgrößen der Sekundärphase bzw. Dispersoide sind bevorzugt nicht deutlich größer als die Korngrößen der Zirkonoxidmatrix. Sie betragen vorzugsweise 0,2 bis 2,0 µm und besonders bevorzugt 0,2 bis 0,5 µm. Der Volumenanteil der Sekundärphase bzw. Dispersoide ist deutlich geringer als der Anteil des Zirkonoxids. Er beträgt bis zu 49 Vol.-%, vorzugsweise 1 bis 10 Vol.-% und besonders bevorzugt 4 bis 6 Vol.-% des Gesamtvolumens. Die Sekundärphase ist chemisch stabil und geht während der Herstellung des Verbundwerkstoffs durch Sintern bei hohen Temperaturen nicht im Zirkonoxid in Lösung.

Eine Sekundärphase ist Strontiumhexaaluminat (SrAl₁₂O₁₉). Als weitere Sekundärphasen bzw. Dispersoide können folgende Verbindungen eingesetzt werden: Lanthanaluminat (LaAl₁₁O₁₈), Hydroxylapatit (Ca₁₀(PO₄)₆(OH)₂), Fluorapatit·(Ca₁₀(PO₄)₆F₂), Tricalciumphoshat (Ca₃(PO₄)₂), Spinell (MgAl₂O₄), Aluminiumoxid (Al₂O₃), Yttrium-Aluminium-Granat (Y₃Al₅O₁₂), Mullit (Al₆Si₂O₃), Zirkon (ZrSiO₄), Quarz (SiO₂), Talk (Mg₃Si₄O₁₀(OH)₂), Kaolinit (Al₂Si₂O₅(OH)₄), Pyrophyllit (Al₂Si₄O₁₀(OH)₂), Kaliumfeldspat (KAlSi₃O₈), Leuzit (KAlSi₂O₆) und Lithiummetasilikat (Li₂SiO₃). Bevorzugt werden Lanthanaluminat, Hydroxylapatit, Fluorapatit, Spinell, Aluminiumoxid und Zirkon; besonders bevorzugt werden Lanthanaluminat, Fluorapatit, Spinell und Aluminiumoxid.

Die Sekundärphase bzw. Dispersoide können inelastische Mikrodeformationen auf mikroskopischer Ebene ermöglichen. Sie können infolge ihrer Kristallstruktur Scherdeformationen auf mikroskopischer Ebene ermöglichen.

Die Sekundärphase wird gemäß einer besonders bevorzugten Ausführungsform der Erfindung nicht erst beim Sintern gebildet, sondern ist Teil der Ausgangssubstanzen, die für die Herstellung der Keramik verwendet werden.

Die Bruchfestigkeit des Verbundwerkstoffs liegt bevorzugt bei ≥ 800 MPa.

Es hat sich überraschend gezeigt, dass die Sekundärphase bzw. Dispersoide die Härte des Verbundwerkstoffes deutlich senken können. Ebenfalls hat sich überraschend gezeigt, dass die Art des chemischen Stabilisators einen deutlichen Einfluss auf die Härte des Verbundwerkstoffs hat.

Des Weiteren hat sich überraschend gezeigt, dass die Risszähigkeit, die Härte und die Schädigungstoleranz des Verbundwerkstoffs durch die Art und die Menge der Sekundärphase bzw. Dispersoide sowie durch die Art des chemischen Stabilisators beeinflusst werden.

Nachfolgend wird die vorliegende Erfindung anhand von Versuchsreihen erläutert, ohne sie dadurch einzuschränken:

### Versuchsreihe 1: Härte in Abhängigkeit vom chemischen Stabilisator

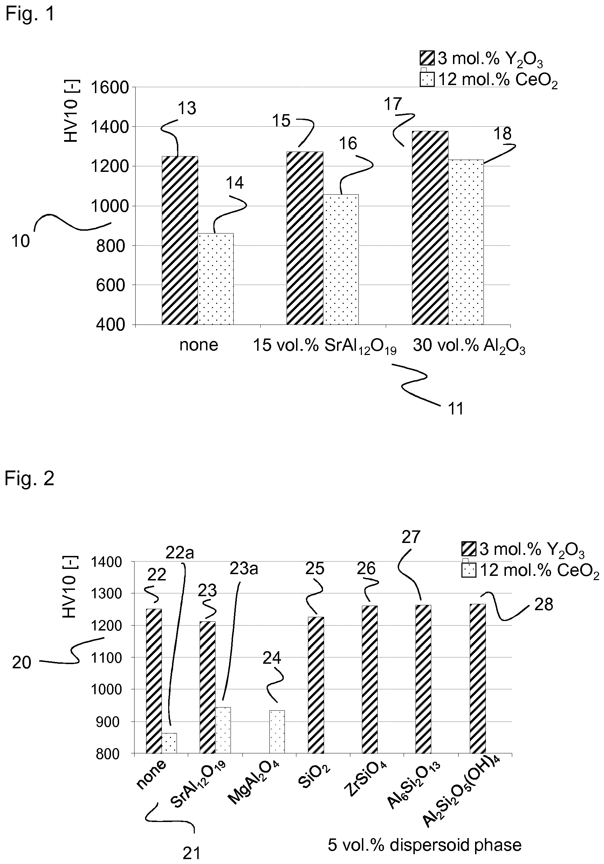

Figur 1 zeigt die Ergebnisse einer Versuchsreihe mit und ohne erfindungsgemäße Dispersoide und jeweils mit unterschiedlichen chemischen Stabilisatoren. Auf der x-Achse 11 ist die Menge und die Art der vergewendeten dispersoiden Phase, nämlich Zirkonoxid ohne Dispersoide mit Y₂O₃-Stabilisierung 13 bzw. CeO₂-Stabilisierung 14, Zirkonoxid mit 15 Vol.-% Strontiumhexaaluminat-Dispersoiden und Y₂O₃-Stabilisierung 15 bzw. CeO₂-Stabilisierung 16 und Zirkonoxid mit 30 Vol.-% Al₂O₃-Dispersoiden und Y₂O₃-Stabilisierung 17 bzw. CeO₂-Stabilisierung 18 gegen die Vickershärte HV 10 auf der y-Achse 10 dargestellt.

Getestet wurden die chemischen Stabilisatoren Yttriumoxid (Y₂O₃) und Ceroxid (CeO₂). Dabei hat sich überraschend gezeigt, dass alle Varianten mit Ce-Stabilisierung 14, 16, 18 deutlich geringere Härtewerte im Vergleich zu Varianten mit Y-Stabilisierung 13, 15, 17 aufweisen. Die Härte wurde mittels Vickerseindruck (HV10) mit einer Kraft von 98,07 N ermittelt.

Reines Ce-stabilisertes Zirkonoxid weist mit 800 (HV10) die geringste Härte auf. In Hinblick auf die erfindungsgemäße Anwendung im dentalen Bereich werden geringere Härten gewünscht. Im molaren Zahnbereich kann ein künstlicher Zahnersatz aus häufig verwendetem Y-TZP auf einen natürlichen Zahn treffen. Die Härte von Y-TZP liegt bei ca. 1250 (HV10). Der natürliche Zahn bzw. das Enamel besitzt aufgrund der eingelagerten Hydroxylapatitkristalle eine deutlich geringere Härte von ca. 400 (HV10). Diese Härtedifferenz kann z.B. bei einer stressbedingten Knirschbewegung (Bruxismus) zu einem erheblichen Abrieb des natürlichen Zahns führen. Aus diesem Grund sind geringere Härten des erfindungsgemäßen Verbundwerkstoffs zielführend. Des Weiteren könnte eine geringere Härte des Verbundwerkstoffs zu einer schädigungsfreien Hartbearbeitung (z.B. beim Einschleifen des künstlichen Zahns im Artikulator) führen.

### Versuchsreihe 2: Härte in Abhängigkeit von der Art der dispersoiden Phase

Figur 2 zeigt eine Versuchsreihe mit unterschiedlichen Dispersoiden im erfindungsgemäßen Verbundwerkstoff. In der Versuchsreihe wurden nur Varianten miteinander verglichen, die denselben Anteil (5 Vol.-%) an dispersoider Phase im Verbundwerkstoff besitzen. Auf der y-Achse 20 ist die Vickershärte HV 10 angegeben. Die x-Achse 21 zeigt Zirkonoxid jeweils mit 5 Vol.-% Dispersoiden (SrAl₁₂O₁₉ 23, 23a, MgAl₂O₄ 24, 24a, SiO₂ 25, ZrSiO₄ 26, Al₆Si₂O₁₃ 27, Al₂Si₂O₅(OH)₄ 28) und zum Vergleich ohne Dispersoide 22, 22a. Für alle Zusammensetzungen wurden Varianten mit Y₂O₃-Stabilisierung 22, 23, 24, 25, 26, 27 und 28 getestet, für die Zirkonoxid-Verbundwerkstoffe mit Strontiumhexaaluminat und Spinell als Dispersoide wurden darüber hinaus Varianten mit CeO₂-Stabilisierung 22a, 23a getestet.

Es hat sich überraschend gezeigt, dass sich die Härte durch die Zugabe von Dispersoiden beeinflussen lässt. Dabei hat sich ebenfalls gezeigt, dass die Einflussgröße von der Zusammensetzung der dispersoiden Phase abhängt.

### Versuchsreihe 3: Härte in Abhängigkeit vom Gehalt an dispersoider Phase

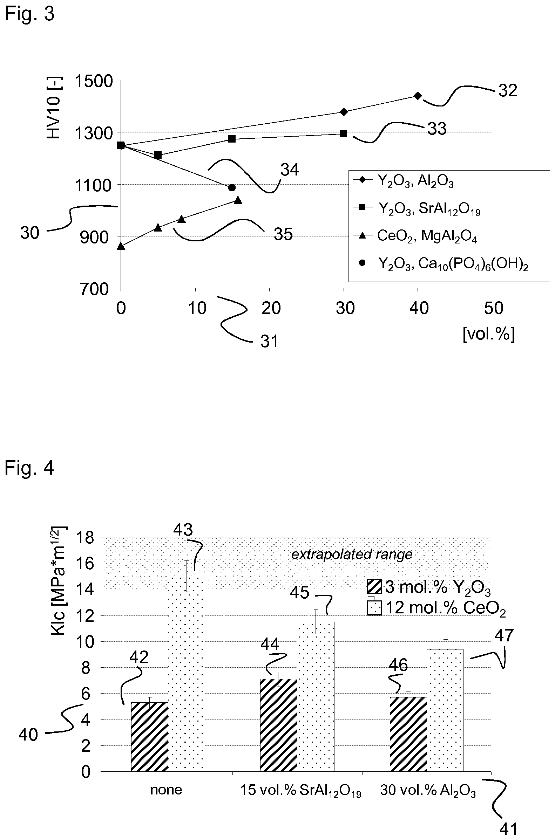

Figur 3 zeigt mehrere Versuchsreihen mit erfindungsgemäßen Dispersoiden und unterschiedlichen Gehalten (Vol.-%) der dispersoiden Phasen (x-Achse 31). Getestet wurden Aluminiumoxid (Al₂O₃) 32, Hydroxylapatit (Ca₁₀(PO₄)₆(OH)₂) 34 und Strontiumhexaaluminat (SrAl₁₂O₁₉) 33 in Y-stabilisiertem Zirkonoxid und Spinell (MgAl₂O₄) 35 in Ce-stabilisiertem Zirkonoxid.

Es hat sich dabei überraschend herausgestellt, dass die Vickershärte, abgetragen auf der y-Achse 30, deutlich durch den Gehalt der dispersoiden Phase beeinflusst werden kann. Die Härte des Verbundwerkstoffs ergibt sich gewöhnlich aus der prozentualen Mischung der Einzelhärten der beteiligten Komponenten. In der Versuchsreihe hat sich überraschend gezeigt, dass diese Mischungsregel nicht immer gilt. Durch das Einbringen von 15 Vol.-% Hydroxylapatit 34 in Y-stabilisiertes Zirkonoxid kann die Härte von ca. 1250 auf ca. 1050 (HV10) gesenkt werden. Durch das Einbringen von 5 Vol.-% Strontiumaluminat 33 kann die Härte von Y-stabilisiertem Zirkonoxid von 1250 auf 1210 (HV10) gesenkt werden.

### Versuchsreihe 4: Risszähigkeit in Abhängigkeit vom chemischen Stabilisator

Figur 4 zeigt eine Versuchsreihe, die den Einfluss des chemischen Stabilisators auf den reinen Zirkonoxidwerkstoff und den erfindungsgemäßen Verbundwerkstoff darstellt. Auf der x-Achse (41) ist der Werkstoff mit der jeweiligen dispersoiden Phase angegenben, nämlich reines Zirkonoxid 42, 43, Zirkonoxid mit 25 Vol.-% Strontiumhexaaluminat als dispersoider Phase 44, 45 und Zirkonoxid mit 30 Vol.-% Al₂O₃ als dispersoider Phase 46, 47, jeweils mit Y₂O₃ 42, 44, 46 oder mit CeO₂ 43, 45, 47 als chemischen Stabilisatoren.

Es konnte überraschend gezeigt werden, dass die Verwendung von Ceroxid (CeO₂) als chemischer Stabilisator die Risszähigkeit (y-Achse 40) des reinen Werkstoffs 43 und des Verbundwerkstoffs 45, 47 deutlich erhöht. Die Risszähigkeit der erfindungsgemäßen Varianten wurde am Vickershärteeindruck (HV10) ermittelt. Hochzähe Varianten wie z.B. reines Ce-stabilisiertes Zirkonoxid 43 zeigten keine Risse am Härteeindruck. Aus diesem Grund wurde in Fig. 4 ein Risszähigkeitswert von 15 MPa*m^{0,5} durch Extrapolation für die hochzähe Variante angenommen.

### Versuchsreihe 5: Risszähigkeit in Abhängigkeit von der dispersoiden Phase

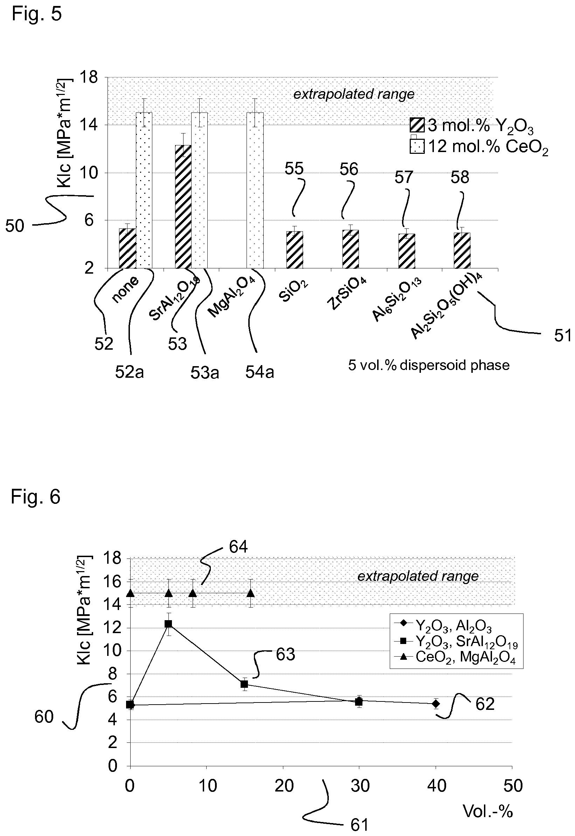

Figur 5 zeigt eine Versuchsreihe mit unterschiedlichen Dispersoiden und deren Einfluss auf die Risszähigkeit (y-Achse 50) des erfindungsgemäßen Verbundwerkstoffs. Auf der x-Achse 51 sind der reine Zirkonoxidwerkstoff 52, 52a, Zirkonoxid mit Strontiumhexaaluminat 53, 53a, Zirkonoxid mit Spinell 54a, Zirkonoxid mit Quarz 55, Zirkonoxid mit Zirkon 56, Zirkonoxid mit Mullit 57 und Zirkonoxid mit Kaolinit 58 als dispersoider Phase, teilweise mit Y₂O₃-Stabilisierung 52, 53, 55, 56, 57, 58 und teilweise mit CeO₂-Stabilisierung 52a, 53a, 54a aufgetragen.

Es hat sich überraschend gezeigt, dass sich durch die Zugabe von dispersoiden Phasen zum Ce-stabilisierten Verbundwerkstoff kein Einfluss auf die Risszähigkeit ergab.

Dagegen hat sich überraschend gezeigt, dass die Zugabe von dispersoiden Phasen zum Y-stabilisierten Verbundwerkstoff teilweise einen deutlichen Einfluss auf die Risszähigkeit ergab. Durch die Zugabe von Strontiumaluminat (SrAl₁₂O₁₉) als dispersoide Phase zum Verbundwerkstoff 53 konnte die Risszähigkeit deutlich von 5,3 auf 12,3 MPa*m^{0,5} erhöht werden.

### Versuchsreihe 6: Risszähigkeit in Abhängigkeit vom Gehalt der dispersoiden Phase

Figur 6 zeigt mehrere Versuchsreihen des erfindungsgemäßen Verbundwerkstoffs mit Dispersoiden und unterschiedlichen Gehalten der dispersoiden Phasen. Auf der x-Achse 61 sind die Gehalte an dispersoider Phase in Vol.-% angegeben. Auf der y-Achse 60 ist die Risszähigkeit in MPa*m^{0,5} aufgetragen.

Getestet wurden Aluminiumoxid (Al₂O₃) 62 und Strontiumaluminat (SrAl₁₂O₁₉) 63 in Y-stabilisiertem Zirkonoxid und Spinell (MgAl₂O₄) 64 in Ce-stabilisiertem Zirkonoxid. Es hat sich überraschend gezeigt, dass abhängig vom verwendeten Dispersoid ein Optimum für den Gehalt der dispersoiden Phase im erfindungsgemäßen Verbundwerkstoff hinsichtlich einer guten Risszähigkeit existiert. Das Optimum für Strontiumaluminat als dispersoide Phase im erfindungsgemäßen Verbundwerkstoff liegt zwischen 1 und 15 Vol.-%.

### Versuchsreihe 7: Schädigungstoleranz in Abhängigkeit vom Gehalt der dispersoiden Phase

Fig. 7 zeigt erfindungsgemäße Verbundwerkstoffe mit einer Y-Stabilisierung und Strontiumhexaaluminat als Sekundärphase. Auf der x-Achse 71 sind die verschiedenen Verbundwerkstoffe anhand ihrer Sekundärphasen-Gehalte in Vol.-% charakterisiert. Auf der y-Achse 70 wurde die Restfestigkeit der Verbundwerkstoffe nach HV50-Schädigung in MPa abgetragen. Die getesten Verbundwerkstoffe sind mit dem Bezugszeichen 72 versehen.

Es zeigt sich deutlich, dass Verbundwerkstoffe 72 mit einem Hexaaluminat-Gehalt zwischen 5 und 15 Vol.-% und insbesondere mit 5 Vol.-% Sekundärphase die Restfestigkeit des Verbundwerkstoffs um ein Vielfaches ansteigt, im Vergleich zu den anderen getesten Werkstoffen.

### Versuchreihe 8: Schädigungstoleranz des Verbundwerkstoffs im Vergleich zu Werkstoffen der Stand der Technik

Fig. 8 zeigt die Restfestigkeitswerte nach unterschiedlichen Schädigungen (hier Vickershärte-Eindrücke) von unterschiedlichen Werkstoffsystemen, einem ZTA-(zirconia toughened alumina) 82, einem Y-TZP- (Y-stabilisiertes polykristallines Zirkonoxid) 83 und einem erfindungsgemäßen Verbundwerkstoff (strontiumhexaaluminate toughened zirconia) 84 . Die geprüfte Eindrucklast ist logarithmisch in N auf der x-Achse 81 gegen die Restfestigkeit in MPa auf der y-Achse 80 aufgetragen.

Im Vergleich zu Werkstoffen aus dem Stand der Technik zeigt sich, dass der neue Verbundwerkstoff bei gleichbleibender Ausgangfestigkeit signifikant höhere Schädigungstoleranzen nach unterschiedlichen Schädigungslasten zeigt.

Nachfolgend sind nochmals die Vorteile des erfindungsgemäßen Verbundwerkstoffs zusammengefasst:
- Herstellung des erfindungsgemäßen Verbundwerkstoffs erfolgt mittels bekannter, konventioneller Keramiktechnologie
- 3-stufige Sinterung (Vorbrand, HIP, Weißbrand), dadurch resultierende höhere Festigkeit
- keine hydrothermale Alterung durch die Verwendung von CeO₂ als chemische Stabilisierung
- deutlich verringerte hydrothermale Alterung durch geringere Anteile an Y₂O₃ als chemische Stabilisierung infolge der mechanischen Teilstabilisierung oder der zusätzlichen Addition von anderen chemischen Stabilisatoren, die keinen negativen Effekt auf die Alterungsbeständigkeit zeigen
- hohe Schädigungstoleranz
- risikoärmere Hartbearbeitung
- geringere Härte
- Verwendung des erfindungsgemäßen Verbundwerkstoffs im zahntechnischen bzw. dentalen Bereich zur Herstellung von Blanks bzw. Blöcken für die CAD/CAM-Bearbeitung im vorgesinterten oder dichtgesinterten Zustand, für Zahnersatz, Zahnrestauration (Brücken, Kronen, Inlays, Onlays)
- bevorzugte Verwendung als Zahnwurzelstift, Implantat, Abutment und weitere Anwendungen
- besonders bevorzugt als Wirbelsäulenimplantat (z.B. Spacer/Cage)

## Patentansprüche

1. Verbundwerkstoff, umfassend eine keramische Matrix aus Zirkonoxid und darin dispergiert zumindest eine Sekundärphase, **dadurch gekennzeichnet, dass** die Matrix aus Zirkonoxid einen Anteil von mindestens 51 Vol.-% am Verbundwerkstoff ausmacht, und dass die Sekundärphase einen Anteil von 1 bis 49 Vol.-% am Verbundwerkstoff ausmacht, wobei das Zirkonoxid zu 90 bis 99%, bevorzugt zu 95 bis 99% bezogen auf den Gesamtzirkonoxid-Anteil in der tetragonalen Phase vorliegt, und chemisch stabilisiert ist und als chemischer Stabilisator Y₂O₃ enthalten ist, wobei der Gesamtgehalt an Y₂O₃ ≤ 3 mol-%, bezogen auf den Zirkonoxidgehalt ist und wobei das Zirkonoxid eine Korngröße von durchschnittlich 0,1 bis 2,0 µm aufweist, und die Sekundärphase aus Strontiumhexaaluminat (SrAl₁₂O₁₉) ist.

2. Verbundwerkstoff nach einem der vorstehenden Ansprüche, **dadurch gekennzeichnet, dass** das Zirkonoxid und/oder die Sekundärphase lösliche Bestandteile enthält.

3. Verbundwerkstoff nach Anspruch 2, **dadurch gekennzeichnet, dass** als lösliche Bestandteile ein oder mehrere der folgenden Elemente, vorzugsweise als Oxid, enthalten sind: Cr, Fe, Mg, Ca, Ti, Y, Ce, Lanthaniden und/oder V.

4. Verbundwerkstoff gemäß einem der vorstehenden Ansprüche, **dadurch gekennzeichnet, dass** die Partikelgrößen der Sekundärphase kleiner oder gleich der Korngrößen des Zirkonoxids sind, wobei die Partikelgrößen vorzugsweise 0,2 bis 2,0 µm, besonders bevorzugt 0,2 bis 0,5 µm betragen.

5. Verbundwerkstoff nach einem der vorstehenden Ansprüche, **dadurch gekennzeichnet, dass** der Volumenanteil der Sekundärphase 1 bis 10 Vol.-%, besonders bevorzugt 4 bis 6 Vol.-% beträgt.

6. Verwendung des Verbundwerkstoffs nach einem der vorstehenden Ansprüche in der Dental- und Medizintechnik, insbesondere zur Herstellung von künstlichem Zahnersatz, Zahnrestaurationen wie Brücken, Kronen, Inlays, und Onlays, Zahnwurzelstiften, Implantaten und Abutments, bevorzugt im Bereich der Implantattechnik, besonders bevorzugt im Wirbelsäulenbereich z.B. Spacer/Cage.

7. Verfahren zur Herstellung eines Sinterformkörpers aus einem Verbundwerkstoff nach einem der Ansprüche 1 bis5, umfassend die Schritte:
a) Pulvermischung gemäß vorgegebener Zusammensetzung in Wasser ansetzen; ggf. Verwendung von Verflüssigern
b) Homogenisierung im Dissolver (schnelllaufender Rührer)
c) Mahlen in Rührwerkskugelmühle,
d) ggf. Zugabe von organischen Bindern
e) Sprühtrocknen,
f) Befeuchten des Granulats mit Wasser und ggf. weiteren Presshilfsmitteln
g) Axialpressen von Blöcken
h) Spanabhebende Bearbeitung von Blöcken im Grün- oder vorgesinterten Zustand,
i) Sinterung,
j) Hartbearbeitung.

## Claims

1. Composite material comprising a zirconia ceramic matrix and at least one secondary phase dispersed therein, **characterized in that** the zirconia matrix represents a proportion of at least 51 vol.% of the composite material, and **in that** the secondary phase represents a proportion of from 1 to 49 vol.% of the composite material, with 90 to 99%, preferably 95 to 99% of the zirconia, based on the total zirconia proportion, being present in the tetragonal phase, and the zirconia being chemically stabilized, and Y₂O₃ being contained as a chemical stabilizer, the total amount of Y₂O₃ being ≤ 3 mol.%, based on the zirconia content, and the zirconia having a grain size of on average from 0.1 to 2.0 µm, and the secondary phase being strontium hexaaluminate (SrAl₁₂O₁₉).

2. Composite material according to the preceding claim, **characterized in that** the zirconia and/or the secondary phase contains soluble components.

3. Composite material according to claim 2, **characterized in that** one or more of the following elements, preferably as an oxide, are contained as soluble components: Cr, Fe, Mg, Ca, Ti, Y, Ce, lanthanides and/or V.

4. Composite material according to any of the preceding claims, **characterized in that** the particle sizes of the secondary phase are less than or equal to the grain sizes of the zirconia, the particle sizes preferably being from 0.2 to 2.0 µm, particularly preferably from 0.2 to 0.5 µm.

5. Composite material according to any of the preceding claims, **characterized in that** the volume proportion of the secondary phase is from 1 to 10 vol.%, particularly preferably from 4 to 6 vol.%.

6. Use of the composite material according to any of the preceding claims in dental and medical engineering, in particular for the production of artificial dentures, tooth restorations such as bridges, crowns, inlays and onlays, dental posts, implants and abutments, preferably in the field of implant technology, particularly preferably in the spinal region, e.g. in a spacer/cage.

7. Method for producing a sintered molded body from a composite material according to any of claims 1 to 5, comprising the steps of:
a) placing a powder mixture according to a predefined composition in water, optionally using liquefiers;
b) carrying out homogenization in the dissolver (high-speed agitator);
c) carrying out grinding in a stirred ball mill;
d) optionally adding organic binders;
e) carrying out spray drying;
f) moistening the granulate using water and optionally further pressing aids;
g) axially pressing blocks;
h) machining blocks in a material-removal process in the green or pre-sintered state;
i) carrying out sintering;
j) carrying out hard machining.

## Revendications

1. Matériau composite comprenant une matrice céramique constituée d'oxyde de zirconium et au moins une phase secondaire dispersée dans celle-ci, **caractérisé en ce que** la matrice constituée d'oxyde de zirconium représente une proportion d'au moins 51 % en volume du matériau composite, et **en ce que** la phase secondaire représente une proportion de 1 à 49 % en volume du matériau composite, l'oxyde de zirconium étant présent en une proportion de 90 à 99 %, de préférence de 95 à 99 %, par rapport à la proportion totale d'oxyde de zirconium dans la phase tétragonale, et étant stabilisé chimiquement, et du Y₂O₃ étant contenu comme stabilisant chimique, la teneur totale en Y₂O₃ étant ≤ 3 % en moles par rapport à la teneur en oxyde de zirconium et l'oxyde de zirconium possédant une taille de grain moyenne de 0,1 à 2,0 µm, et la phase secondaire étant constituée d'hexaaluminate de strontium (SrAl₁₂O₁₉).

2. Matériau composite selon l'une des revendications précédentes, **caractérisé en ce que** l'oxyde de zirconium et/ou la phase secondaire contiennent des constituants solubles.

3. Matériau composite selon la revendication 2, **caractérisé en ce qu'**un ou plusieurs des éléments suivants, de préférence sous forme d'oxyde, sont contenus comme constituants solubles : Cr, Fe, Mg, Ca, Ti, Y, Ce, des lanthanides et/ou V.

4. Matériau composite selon l'une des revendications précédentes, **caractérisé en ce que** les tailles de particule de la phase secondaire sont inférieures ou égales aux tailles de grain de l'oxyde de zirconium, les tailles de particule étant de préférence de 0,2 à 2,0 µm, de manière particulièrement préférée de 0,2 à 0,5 µm.

5. Matériau composite selon l'une des revendications précédentes, **caractérisé en ce que** la proportion volumique de la phase secondaire est de 1 à 10 % en volume, de manière particulièrement préférée de 4 à 6 % en volume.

6. Utilisation du matériau composite selon l'une des revendications précédentes dans la technologie dentaire et médicale, en particulier pour la production de prothèses dentaires artificielles, de restaurations dentaires telles que des ponts, des couronnes, des incrustations et des appositions, des tenons radiculaires dentaires, des implants et des piliers, de préférence dans le domaine de la technologie des implants, de manière particulièrement préférée dans la région de la colonne vertébrale, par exemple pour des espaceurs/cages.

7. Procédé de production d'un corps moulé fritté à partir d'un matériau composite selon l'une des revendications 1 à 5, comprenant les étapes :
a) de préparation dans de l'eau d'un mélange pulvérulent selon la composition spécifiée ; d'utilisation éventuelle de fluidifiants,
b) d'homogénéisation dans le dissolveur (agitateur à grande vitesse),
c) de broyage dans un broyeur agitateur à boulets,
d) d'ajout éventuel de liants organiques,
e) de séchage par pulvérisation,
f) d'humidification du granulé avec de l'eau et éventuellement d'autres agents auxiliaires de pressage,
g) de pressage axial de blocs,
h) d'usinage de blocs par enlèvement de copeaux à l'état cru ou pré-fritté,
i) de frittage,
j) d'usinage dur.
